# EUROPEAN PATENT SPECIFICATION

(11) **EP 3 560 346 B1**
(45) Date of publication and mention of the grant of the patent: **22.09.2021**
(21) Application number: 17883746.4
(22) Date of filing: 18.12.2017
(51) Int. Cl.: A61K 36/48, A61K 38/01, A61K 38/12, A61K 38/16, A23F 3/16, A23L 2/52, A23L 33/105, A23F 3/26, A23F 3/30, A61K 31/353, A61K 36/82, A23L 33/18

(54) **FOOD AND BEVERAGES CONTAINING EPIGALLOCATECHIN GALLATE AND CYCLO(PROLYL-THREONINE)**
NAHRUNGSMITTEL UND GETRÄNKE MIT EPIGALLOCATECHINGALLAT UND CYCLO(PROLYL-THREONIN)
ALIMENTS ET BOISSONS CONTENANT DU GALLATE D'ÉPIGALLOCATÉCHINE ET DE LA CYCLO(PROLYL-THRÉONINE)

(30) Priority: 21.12.2016 JP 2016247631
(43) Date of publication of application: 30.10.2019
(73) Proprietor: Suntory Holdings Limited, Kita-ku, Osaka-shi Osaka 530-8203 (JP)
(72) Inventor: YAMAMOTO, Kenji, Soraku-gun Kyoto 619-0284 (JP); MATSUBAYASHI, Hideki, Kawasaki-shi Kanagawa 211-0067 (JP)
(74) Representative: Vossius & Partner Patentanwälte Rechtsanwälte mbB
(86) International application number: PCT/JP2017/045249
(87) International publication number: WO 2018/117002

(56) References cited:
- EP-A1- 1 297 749
- EP-A1- 1 361 798
- WO-A1-2014/200000
- KENJI YAMAMOTO ET AL: "Development of LC-MS/MS analysis of cyclic dipeptides and its application to tea extract", BIOSCIENCE, BIOTECHNOLOGY AND BIOCHEMISTRY, vol. 80, no. 1, 24 August 2015 (2015-08-24), pages 172-177, XP055495546, JP ISSN: 0916-8451, DOI: 10.1080/09168451.2015.1075865
- YAMAMOTO, K. et al.: "Development of LC-MS/MS analysis of cyclic dipeptides and its application to tea extract", Biosci. Biotech. Biochem., vol. 80, no. 1 24 August 2015 (2015-08-24), pages 172-177, XP055495546, ISSN: 0916-8451 Retrieved from the Internet: URL:https://doi.org/10.1080/09168451.2015. 1075865
- LIN, J. K. et al.: "Survey of Catechins, Gallic Acid, and Methylxanthines in Green, Oolong, Pu-erh, and Black Teas", J. Agric. Food Chem., vol. 46, no. 9, 1998, pages 3635-3642, XP055024257,
- KISHI, HIROKO et al.: "Determination of Catechins, Caffeine and Theanine in Chinese Tea Bought by Internet", Bull. Kanagawa Ins. of P. H., vol. 35, 2005, pages 30-32, XP009515226,

## Description

### TECHNICAL FIELD

The present invention relates to a food or beverage product containing epigallocatechin gallate and cycloprolyl threonine. More specifically, it relates to a food or beverage product wherein a content of epigallocatechin gallate falls within a specific range and a content of cycloprolyl threonine falls within a specific range, a method for producing the food or beverage product, and a method for reducing lingering unpleasant astringent taste of epigallocatechin gallate in the food or beverage product.

### BACKGROUND ART

It has been reported that catechins such as epigallocatechin gallate have physiological activities such as inhibitory activity against cholesterol elevation and inhibitory activity against α-amylase activity (PTLs 1 and 2). Other than above, various physiological activities such as antioxidant activity, bactericidal activity, anticancer activity, hypotensive activity, and suppression of blood sugar rise are known, and there is a growing need for a beverage which allows effectively intake of catechins (PTL 3).

Meanwhile, consumers' preferences for beverages vary widely, and there is recently a tendency that beverages having enhanced drinkability are preferred. Beverages with a high content of catechins are beverages which can be expected to have the physiological activities of catechins, but bitterness and astringency are strong, and they are not suitable for being taken in a large amount at once. Therefore, in order to enhance the drinkability, the content of catechins needs to be suppressed to a low level. However, in this case, beverages have reduced taste and become watery, and they are not satisfactory in terms of taste.

### CITATION LIST

### PATENT LITERATURE

PTL 1: Japanese Patent Laid-Open No. 60-156614
PTL 2: Japanese Patent Laid-Open No. 3-133928
PTL 3: Japanese Patent Laid-Open No. 2001-97968

### SUMMARY OF INVENTION

### TECHNICAL PROBLEM

It is an object of the present invention to provide a food or beverage product having good taste with the lingering unpleasant astringent taste of epigallocatechin gallate reduced.

### SOLUTION TO PROBLEM

As a result of diligent studies in order to solve the above problems, the inventors have focused on use of cycloprolyl threonine that is one of cyclic dipeptides. First, the inventors have found that, in the case of using cycloprolyl threonine alone in a food or beverage product, favorable intake is impossible due to excessively strong astringent taste. However, the inventors have found surprisingly that the lingering unpleasant astringent taste of epigallocatechin gallate can be reduced, with no astringent taste of cycloprolyl threonine sensed, by adding a certain amount of cycloprolyl threonine to a food or beverage product containing a certain amount of epigallocatechin gallate, thereby accomplishing the present invention.

That is, the present invention is defined in the claims.

### ADVANTAGEOUS EFFECTS OF INVENTION

According to the present invention, a food or beverage product having good taste, with lingering unpleasant astringent taste unique to epigallocatechin gallate and astringent taste unique to cycloprolyl threonine in the food or beverage product reduced, can be obtained.

### DESCRIPTION OF EMBODIMENTS

### 1. Food or beverage product

One embodiment of the present invention is a food or beverage product comprising epigallocatechin gallate and cycloprolyl threonine, wherein a content of epigallocatechin gallate falls within a specific range and a content of cycloprolyl threonine falls within a specific range.

### 1-1. Epigallocatechin gallate

Epigallocatechin gallate is one of flavonoids. In particular, (-)-epigallocatechin gallate is a main polyphenol contained in leaves of green tea plants, Camellia Sinesis, and is known to have various physiological activities such as antioxidant activity, antimutagenic activity, antibacterial activity, and antiallergic activity.

The content of epigallocatechin gallate in the food or beverage product of the present invention is not specifically limited, but when the content of epigallocatechin gallate is excessively large, it may be difficult to obtain the effect of reducing the lingering unpleasant astringent taste of epigallocatechin gallate by cycloprolyl threonine in some cases. The lower limit of the content of epigallocatechin gallate in the food or beverage product of the present invention is 0.4 mg or more, preferably 0.5 mg or more, more preferably 0.9 mg or more, furthermore preferably 1.0 mg or more, with respect to 100 mL of the food or beverage product. Further, the upper limit of the content of epigallocatechin gallate in the food or beverage product of the present invention is 16 mg or less, preferably 15 mg or less, more preferably 11 mg or less, furthermore preferably 10 mg or less, particularly preferably 8.0 mg or less, with respect to 100 mL of the food or beverage product. Typically, the content range of epigallocatechin gallate in the food or beverage product of the present invention is 0.4 to 16 mg, preferably 0.5 to 15 mg, more preferably 0.9 to 11 mg, furthermore preferably 1.0 to 10 mg, particularly preferably 1.0 to 8.0 mg, with respect to 100 mL of the food or beverage product.

The content of epigallocatechin gallate can be measured by a known method such as HPLC.

The epigallocatechin gallate used in the present invention is not particularly limited by the form, the production method, or the like, but the epigallocatechin gallate can be produced, for example, by extraction and purification from green tea leaves according to the method disclosed in Japanese Patent Application Laid-Open No. 2001-97968. Alternatively, a crudely purified product of epigallocatechin gallate may be used in the present invention. This includes extracts from tea leaves, preferably, green tea leaves and crushed products of such tea leaves. Further, such extracts include tea beverages, particularly, green tea beverages.

Further, epigallocatechin gallate is one of catechins. The term "catechins" is a generic term, including catechin, epicatechin, gallocatechin, epigallocatechin, catechin gallate, epicatechin gallate, and gallocatechin gallate, other than epigallocatechin gallate. In the food or beverage product of the present invention, the contents of other catechins are not specifically limited, as long as the content of epigallocatechin gallate satisfies the aforementioned ranges.

In the present invention, the method for adjusting the content of epigallocatechin gallate is not specifically limited, as long as the content of epigallocatechin gallate in the food or beverage product falls within the aforementioned ranges. For example, commercially available or synthetic products of epigallocatechin gallate can be used, or raw materials (such as tea beverages) containing epigallocatechin gallate can be also used. Further, only one of commercially available or synthetic products of epigallocatechin gallate, or raw materials containing epigallocatechin gallate can be also used, or two or more of them can be also used in combination.

### 1-2. Cycloprolyl threonine

Cycloprolyl threonine in the present invention is one of cyclic dipeptides and is a compound having a diketopiperazine structure formed by dehydration condensation of proline and threonine.

Cycloprolyl threonine in the present invention may be in the form of a pharmacologically acceptable salt (including inorganic salts and organic salts) such as sodium salt, potassium salt, calcium salt, magnesium salt, ammonium salt, hydrochloride, sulfate, nitrate, phosphate, and organic acid salts (such as acetate, citrate, maleate, malate, oxalate, lactate, succinate, fumarate, propionate, formate, benzoate, picrate, benzene sulfonate, and trifluoroacetate) of cycloprolyl threonine, but there is no limitation to these. Such a salt of cycloprolyl threonine can be easily prepared by those skilled in the art using any known method in the field. In this description, "cycloprolyl threonine or a salt thereof' may be collectively referred to simply as "cycloprolyl threonine".

Cycloprolyl threonine used in the present invention can be prepared according to a known method in the field. For example, it may be produced by a chemical synthesis method, an enzymatic method, or a microbial fermentation method, may be synthesized by dehydration and cyclization of linear prolyl threonine, or can be prepared according to the method described in Japanese Patent Application Laid-Open No. 2003-252896 or Journal of Peptide Science, 10, 737-737, 2004. For example, an animal or plant-derived peptide obtained by applying enzymatic treatment or heat treatment to a raw material containing an animal or plant-derived protein is further subjected to high-temperature heat treatment, so that a heat-treated product of the animal or plant-derived peptide rich in cycloprolyl threonine can be obtained. From these viewpoints, cycloprolyl threonine used in the present invention may be chemically or biologically synthesized or may be obtained from an animal or plant-derived peptide.

The content of cycloprolyl threonine in the food or beverage product of the present invention is not specifically limited, but when the content of cycloprolyl threonine is excessively large, favorable intake may be impossible due to excessively strong lingering unpleasant bitterness unique to cycloprolyl threonine. The lower limit of the content of cycloprolyl threonine in the food or beverage product of the present invention is 0.0020 mg or more, preferably 0.0021 mg or more, more preferably 0.0050 mg or more, furthermore preferably 0.0053 mg or more, with respect to 100 mL of the food or beverage product. Further, the upper limit of the content of cycloprolyl threonine in the food or beverage product of the present invention is preferably 0.35 mg or less, more preferably 0.32 mg or less, 0.055 mg or less, furthermore preferably 0.053 mg or less, with respect to 100 mL of the food or beverage product. Typically, the content range of cycloprolyl threonine in the food or beverage product of the present invention is 0.0020 to 0.35 mg, preferably 0.0021 to 0.32 mg, more preferably 0.0050 to 0.055 mg, furthermore preferably 0.0053 to 0.053 mg, with respect to 100 mL of the food or beverage product.

The content of cycloprolyl threonine can be measured by a known method and can be measured, for example, by the LC-MS/MS method.

In the present invention, the method for adjusting the content of cycloprolyl threonine is not specifically limited, as long as the content of cycloprolyl threonine in the food or beverage product falls within the aforementioned ranges. For example, commercially available cycloprolyl threonine, synthetic cycloprolyl threonine that is produced by a chemical synthesis method, an enzymatic method, or a microbial fermentation method, or a heat-treated product of animal or plant-derived peptide rich in cycloprolyl threonine can be used. Further, only one of commercially available or synthetic products of cycloprolyl threonine, and a heat-treated product of animal or plant-derived peptide rich in cycloprolyl threonine can be used, or two or more of them can be used in combination.

A heat-treated product of animal or plant-derived peptide contains a wide variety of cyclic dipeptides other than cycloprolyl threonine. Therefore, in the case of adjusting the content of cycloprolyl threonine in the food or beverage product using such a heat-treated product of animal or plant-derived peptide, cyclic dipeptides other than cycloprolyl threonine are also added in the food or beverage product. However, in the present invention, lingering unpleasant astringent taste unique to epigallocatechin gallate is made remarkable when the content of epigallocatechin gallate in the food or beverage product falls within a predetermined range, and the lingering unpleasant astringent taste of epigallocatechin gallate is reduced by adjusting the content of cycloprolyl threonine in the food or beverage product to a specific range, regardless of the contents of other cyclic dipeptides.

### 1-3. Heat-treated product of animal or plant-derived peptide

In this description, the "animal or plant-derived peptide" is not specifically limited, but soybean peptide, tea peptide, malt peptide, whey peptide, and collagen peptide, for example, can be used therefor. Among these, soybean peptide and tea peptide are preferable in the present invention. An animal or plant-derived peptide prepared from a raw material containing an animal or plant-derived protein or a protein using a known method may be used, or a commercially available animal or plant-derived peptide may be used.

### 1-3-1. Soybean peptide

In this description, "soybean peptide" refers to a low-molecular weight peptide obtained by applying enzymatic treatment or heat treatment to soy protein to lower the molecular weight of the protein. Any soybean (scientific name: Glycine max) can be used as a raw material with no limitation in variety and production area, and processed products such as a crushed product can be also used.

### 1-3-2. Tea peptide

In this description, "tea peptide" refers to a low-molecular weight peptide derived from tea obtained by applying enzymatic treatment or heat treatment to a tea (including tea leaves or used tea leaves) extract to lower the molecular weight of the protein. As the tea leaves serving as a raw material to be extracted, portions that can be extracted for intake such as leaves and stems of tea produced from tea trees (scientific name: Camellia sinensis) can be used. Further, the form such as macrophyllous and powder forms is not limited. The harvest time of tea leaves can be also appropriately selected corresponding to the desired flavor.

### 1-3-3. Malt peptide

In this description, "malt peptide" refers to a low-molecular weight peptide derived from malt obtained by applying enzymatic treatment or heat treatment to an extract obtained from malt or a ground product thereof to lower the molecular weight of the protein. Any malt peptide can be used as a raw material with no limitation in variety and production area, but barley malt obtained by germinating seeds of barley is particularly suitably used therefor. In this description, barley malt may be expressed simply as malt.

### 1-3-4. Whey peptide

The raw material of whey peptide is not specifically limited, but examples thereof include WPC (Whey Protein Concentrate) and WPI (Whey Protein Isolate) which are whey proteins. Whey peptide refers to a product obtained by degrading such a whey protein with an enzyme or the like. The degree of degradation may vary, but when the degree of degradation is low, milk odor tends to be stronger, and the liquid after dissolution tends to be opaque (turbid). Meanwhile, when the degree of degradation is high, the liquid after dissolution tends to be transparent, but bitterness and astringency tend to increase.

### 1-3-5. Collagen peptide

In this description, "collagen peptide" refers to a low-molecular weight peptide obtained by applying enzymatic treatment or heat treatment to collagen or a ground product thereof to lower the molecular weight of collagen. Collagen is a main protein for connective tissues of animals and is the most abundant protein in mammalian bodies including humans.

### 1-3-6. Heat-treated product of animal or plant-derived peptide

As described above, a heat-treated product of animal or plant-derived peptide rich in cycloprolyl threonine can be obtained by applying high-temperature heat treatment to an animal or plant-derived peptide. In this description, the "high-temperature heat treatment" is treatment for a certain time at a temperature of 100°C or more and a pressure over the atmospheric pressure. As a high-temperature and high-pressure treatment device, a pressure resistant extractor, a pressure cooker, an autoclave or the like can be used corresponding to the conditions.

The temperature in the high-temperature heat treatment is not specifically limited, as long as it is 100°C or more, but is preferably 100°C to 170°C, more preferably 110°C to 150°C, furthermore preferably 120°C to 140°C. This temperature is a value obtained by measuring the temperature at the outlet of an extraction column in the case of using a pressure resistant extractor as a heating device and is a value obtained by measuring the temperature at the center of a pressure container in the case of using an autoclave as a heating device.

The pressure in the high-temperature heat treatment is not specifically limited, as long as it is a pressure over the atmospheric pressure, but is preferably 0.101 MPa to 0.79 MPa, more preferably 0.101 MPa to 0.60 MPa, furthermore preferably 0.101 MPa to 0.48 MPa.

The time of the high-temperature heat treatment is not specifically limited, as long as a treated material containing cycloprolyl threonine is obtained, but is preferably about 15 minutes to 600 minutes, more preferably about 30 minutes to 500 minutes, furthermore preferably about 60 minutes to 300 minutes.

Further, the conditions for the high-temperature heat treatment of the animal or plant-derived peptide is not specifically limited, as long as a treated material containing cycloprolyl threonine is obtained, but [temperature:pressure:time] is preferably [100°C to 170°C:0.101 MPa to 0.79 MPa:15 minutes to 600 minutes], more preferably [110°C to 150°C:0.101 MPa to 0.60 MPa:30 minutes to 500 minutes], furthermore preferably [120°C to 140°C:0.101 MPa to 0.48 MPa:60 minutes to 300 minutes].

The heat-treated product of animal or plant-derived peptide obtained may be subjected to treatment such as filtration, centrifugation, concentration, ultrafiltration, freeze drying, and powderization, as needed. Further, if a desired content of specific cycloprolyl threonine in the heat-treated product of animal or plant-derived peptide is not satisfied, other animal or plant-derived peptides, commercially available products, or synthetic products can be appropriately used and added to the specific cycloprolyl threonine that is insufficient.

### 1-4. Relationship between content of epigallocatechin gallate and content of cycloprolyl threonine

In the food or beverage product of the present invention, it is preferable that the content of epigallocatechin gallate be 0.40 to 11 mg/100 mL, the content of cycloprolyl threonine be 0.0020 to 0.055 mg/100 mL, and the content of epigallocatechin gallate (mg/100 mL) (X) and the content of cycloprolyl threonine (mg/100 mL) (Y) satisfy Y ≥ 0.0034 × X^{-1.106} and Y ≥ 0.00009 × X^{1.7421}, for providing a food or beverage product having good taste, with lingering unpleasant astringent taste unique to epigallocatechin gallate and astringent taste unique to cycloprolyl threonine in the food or beverage product reduced.

### 1-5. Type of food or beverage product

One embodiment of the present invention is a food or beverage product, wherein a content of epigallocatechin gallate falls within a specific range and a content of cycloprolyl threonine falls within a specific range.

Preferable ranges of the content of epigallocatechin gallate and the content of cycloprolyl threonine are as mentioned above, and a food or beverage product having good taste, with lingering unpleasant astringent taste unique to epigallocatechin gallate in the food or beverage product reduced, can be obtained by adjusting the content of epigallocatechin gallate and the content of cycloprolyl threonine to the aforementioned ranges.

Further, it has been confirmed that cycloprolyl threonine has GLP-2 secretion-promoting activity and TRPV1-stimulating activity (PCT/JP2016/069084 and PCT/JP2016/070639), and the food or beverage product of the present invention can be a food or beverage product for promoting GLP-2 secretion or stimulating TRPV1. Further, it can be also a food or beverage product with a display of a function relating to promotion of GLP-2 secretion or stimulation of TRPV1.

The type of the food or beverage product of the present invention is not specifically limited, but examples thereof include foods, beverages, food/beverage compositions, food compositions, and beverage compositions. The food or beverage product of the present invention is preferably a beverage. Further, the type of beverage is not specifically limited, and examples thereof include soft drinks, non-alcohol beverages, and alcohol beverages. The beverage may be a beverage free from carbon dioxide gas or may be a beverage containing carbon dioxide gas. Examples of the beverage free from carbon dioxide gas include tea beverages, coffees, fruit juice beverages, milk beverages, and sports drinks, but there is no limitation to these. Examples of the beverage containing carbon dioxide gas include Colas, Diet Colas, ginger ales, ciders, and fruit-flavored carbonated waters, but there is no limitation to these. In particular, the beverage of the present invention is preferably a tea beverage.

The tea beverage in the present invention is a liquid beverage obtained by extracting, with an aqueous solvent, teas such as green tea, black tea, oolong tea, and Pu'er tea produced mainly using leaves and stems of tea trees (scientific name: Camellia sinensis), these teas further blended with brown rice, wheat and barley, and others various plant raw materials, raw materials mainly such as leaves, stems, rhizomes, roots, flowers, and fruits of various plants other than tea trees, or such raw materials blended with each other.

Examples of the tea beverage of the present invention can include unfermented teas (such as green tea), semi-fermented teas (such as oolong tea), and fermented teas (such as black tea), specifically, teas including steamed unfermented teas (green teas) such as sencha, bancha, roasted green tea, gyokuro, kabusecha, and tencha; unfermented teas such as kamairicha, e.g., Ureshino tea, Aoyagi tea, and various Chinese teas; semi-fermented teas such as pouchong tea, Tieguanyin tea, and oolong tea; and fermented teas such as black tea, Awa bancha, and Pu'er tea. Further, the tea leaves are not limited at all, as long as they are portions that can be extracted to be taken, and leaves, stems, and the like can be appropriately used. Further, the form such as macrophyllous and powder forms is not limited.
Preferable examples of the tea beverage of the present invention include oolong tea and black tea.

### 1-6. Other components

The food or beverage product of the present invention may contain various additives corresponding to the type of the food or beverage product other than the various components mentioned above. Examples of the various additives include sweeteners of saccharides other than above, acidulants, perfumes, vitamins, pigments, antioxidants, emulsifiers, preservatives, extracts, dietary fibers, pH adjusters, and quality stabilizers.

### 1-7. Food or beverage product packaged in container

The food or beverage product of the present invention can be packaged in a container after undergoing a step such as sterilization, as required. For example, a method of heat-sterilizing the food or beverage product after being packaged in a container or a method of sterilizing the food or beverage product and thereafter packaging it into a container in a sterile environment can be used.

The type of container is not particularly limited, and any one of containers generally used for food or beverage products such as resin containers including PET bottles, paper containers including paper packs, glass containers including glass bottles, metal containers including aluminum cans and steel cans, and aluminum pouches, for example, can be used.

### 2. Method for producing food or beverage product

According to an embodiment, the present invention is a method for producing a food or beverage product, the method comprising: step (a) of adding epigallocatechin gallate to adjust the content of epigallocatechin gallate in the food or beverage product to 0.40 to 16 mg/100 mL; and step (b) of adding cycloprolyl threonine to adjust the content of cycloprolyl threonine in the food or beverage product to 0.0020 to 0.35 mg/100 mL. Further, the content of epigallocatechin gallate in the food or beverage product adjusted in step (a) above can be also 0.4 to 16 mg/100 mL, 0.5 to 15 mg/100 mL, 0.9 to 11 mg/100 mL, 1.0 to 10 mg/100 mL, or 1.0 to 8.0 mg/100 mL. Further, the content of cycloprolyl threonine in the food or beverage product adjusted in step (b) above can be also 0.0020 to 0.35 mg/100 mL, 0.0021 to 0.32 mg/100 mL, 0.0050 to 0.055 mg/100 mL, or 0.0053 to 0.053 mg/100 mL.

Further, the production method can comprise a step of adjusting the content of epigallocatechin gallate to satisfy 0.40 to 11 mg/100 mL, a step of adjusting the content of cycloprolyl threonine to satisfy 0.0020 to 0.055 mg/100 mL, or a step of adjusting the content of epigallocatechin gallate (mg/100 mL) (X) and the content of cycloprolyl threonine (mg/100 mL) (Y) to satisfy Y ≥ 0.0034 × X^{-1.106} and Y ≥ 0.00009 × X^{1.7421}.

In the method for producing a food or beverage product of the present invention, the method for adjusting the contents of epigallocatechin gallate and cycloprolyl threonine is not specifically limited, and the contents can be adjusted to predetermined ranges, for example, by adding epigallocatechin gallate and cycloprolyl threonine. The method for adding epigallocatechin gallate and cycloprolyl threonine is not specifically limited, and commercially available products or synthetic products of epigallocatechin gallate and cycloprolyl threonine may be added, or raw materials or the like containing epigallocatechin gallate and cycloprolyl threonine may be added, for example. The content ranges or the like of epigallocatechin gallate and cycloprolyl threonine are as mentioned above.

The type of food or beverage product produced in the present invention is not specifically limited, as described above, but the food or beverage product in the present invention is preferably a beverage, and examples thereof include soft drinks, non-alcohol beverages, and alcohol beverages. The beverage may be a beverage free from carbon dioxide gas or may be a beverage containing carbon dioxide gas. Examples of the beverage free from carbon dioxide gas include tea beverages, coffees, fruit juice beverages, milk beverages, and sports drinks, but there is no limitation to these. Examples of the beverage containing carbon dioxide gas include Colas, Diet Colas, ginger ales, ciders, and fruit-flavored carbonated waters, but there is no limitation to these. In particular, the beverage produced in the present invention is preferably a tea beverage.

The method for producing a food or beverage product of the present invention can further comprise the steps of adding an additive or the like, which is generally contained in food or beverage products, and packaging the food or beverage product in a container. The types of additives and container are as described above, and a known method can be used as the packaging method in the container.

In the method for producing a food or beverage product of the present invention, the aforementioned various steps may be performed in any order, as long as the contents and the weight ratio of the food or beverage product finally obtained fall within the predetermined ranges.

### 3. Method for reducing lingering unpleasant astringent taste of epigallocatechin gallate in food or beverage product

According to an embodiment, the present invention is a method for reducing lingering unpleasant astringent taste of epigallocatechin gallate in a food or beverage product, the method comprising: step (a) of adding epigallocatechin gallate to adjust the content of epigallocatechin gallate in the food or beverage product to 0.40 to 16 mg/100 mL; and step (b) of adding cycloprolyl threonine to adjust the content of cycloprolyl threonine in the food or beverage product to 0.0020 to 0.35 mg/100 mL. Further, the content of epigallocatechin gallate in the food or beverage product adjusted in step (a) above can be also 0.4 to 16 mg/100 mL, 0.5 to 15 mg/100 mL, 0.9 to 11 mg/100 mL, 1.0 to 10 mg/100 mL, or 1.0 to 8.0 mg/100 mL. Further, the content of cycloprolyl threonine in the food or beverage product adjusted in step (b) above can be also 0.0020 to 0.35 mg/100 mL, 0.0021 to 0.32 mg/100 mL, 0.0050 to 0.055 mg/100 mL, or 0.0053 to 0.053 mg/100 mL.

Further, the method can comprise a step of adjusting the content of epigallocatechin gallate to satisfy 0.40 to 11 mg/100 mL, a step of adjusting the content of cycloprolyl threonine to satisfy 0.0020 to 0.055 mg/100 mL, or a step of adjusting the content of epigallocatechin gallate (mg/100 mL) (X) and the content of cycloprolyl threonine (mg/100 mL) (Y) to satisfy Y ≥ 0.0034 × X^{-1.106} and Y ≥ 0.00009 × X^{1.1421}.

In the method, the method for adjusting the contents of epigallocatechin gallate and cycloprolyl threonine is not specifically limited, and the contents can be adjusted to predetermined ranges, for example, by adding epigallocatechin gallate and cycloprolyl threonine. The method for adding epigallocatechin gallate and cycloprolyl threonine is not specifically limited, and commercially available products or synthetic products of epigallocatechin gallate and cycloprolyl threonine may be added, or raw materials or the like containing epigallocatechin gallate and cycloprolyl threonine may be added, for example. The content ranges or the like of epigallocatechin gallate and cycloprolyl threonine are as mentioned above.

The type of food or beverage product in the method is not specifically limited, as described above, but the food or beverage product in the present invention is preferably a beverage, and examples thereof include soft drinks, non-alcohol beverages, and alcohol beverages. The beverage may be a beverage free from carbon dioxide gas or may be a beverage containing carbon dioxide gas. Examples of the beverage free from carbon dioxide gas include tea beverages, coffees, fruit juice beverages, milk beverages, and sports drinks, but there is no limitation to these. Examples of the beverage containing carbon dioxide gas include Colas, Diet Colas, ginger ales, ciders, and fruit-flavored carbonated waters, but there is no limitation to these. In particular, the beverage produced in the present invention is preferably a tea beverage.

The aforementioned method can comprise the steps of adding an additive or the like, which is generally contained in food or beverage products and a step of packaging the food or beverage product in a container. The types of additives and container are as described above, and a known method can be used as the packaging method in the container.

In the aforementioned method, the various steps may be performed in any order, as long as the contents and the weight ratio of the food or beverage product finally obtained fall within the predetermined ranges.

### EXAMPLES

Hereinafter, the present invention will be described more specifically based on examples.

### Example 1: Evaluation of influence of content of cycloprolyl threonine on lingering unpleasant astringent taste of epigallocatechin gallate

Sample beverages were prepared, with the content of cycloprolyl threonine and the content of epigallocatechin gallate in the beverages variously changed, and were each subjected to a sensory evaluation test. The method for preparing the sample beverages and the method of the sensory evaluation test are shown below.

### <Sample beverages 1 to 34>

Sample beverages 1 to 34 were prepared by mixing cycloprolyl threonine (stock solution concentration: 1 mg/mL) and epigallocatechin gallate (stock solution concentration: 10 mg/mL) so that the content of cycloprolyl threonine in each sample beverage was 0, 0.0021, 0.0053, 0.021, 0.053, 0.32, or 1.0 mg/100 mL, and the content of epigallocatechin gallate in each sample beverage was 0, 0.1, 0.5, 1, 5, 8, 10, 15, or 20 mg/100 mL. Epigallocatechin gallate used was of standard special grade, available from NACALAI TESQUE, INC., and had a purity of > 98%. Cycloprolyl threonine used was available from Carl Bechem GmbH and had a purity of > 99%.

### <Sample beverages 35 to 38>

Sample beverages 35 to 38 were prepared by adding 50 mg of a heat-treated product of tea peptide (tea extract), 20 mg of a heat-treated product of soybean peptide (soybean extract), 200 mg of a heat-treated product of collagen peptide (collagen extract), or 15 mg of a heat-treated product of whey peptide (whey extract) to each sample beverage, and mixing epigallocatechin gallate with each of the heat-treated product of peptides, so that the content of epigallocatechin gallate in the sample beverage was 5.0 mg/100 mL. The heat-treated products of various peptides were prepared by the following methods.

### (1) Preparation of heat-treated product of soybean peptide

The heat-treated product of soybean peptide used was obtained by treating soybean peptide with heat, followed by freeze drying. The heat-treated product of soybean peptide was produced by high-temperature high-pressure treatment of soybean peptide in a liquid. Specifically, about 15 ml of distilled water was added to 3 g of soybean peptide (Hinute AM, available from FUJI OIL CO., LTD.) and was put into an autoclave (available from TOMY SEIKO CO., LTD.), followed by high-temperature high-pressure treatment at 135°C and 0.31 MPa for 3 hours. Thereafter, it was subjected to freeze drying to obtain a heat-treated product of soybean peptide (soybean extract) in powder form.

### (2) Preparation of heat-treated product of tea peptide

The first-picked tea leaves (variety: Yabukita, and total nitrogen: 6.3%) from Kagoshima was used as a plant. This tea was first subjected to pretreatment for reducing water-soluble proteins (three times of pre-extraction). That is, 200 g of hot water was added to 10 g of tea, followed by appropriate stirring and extraction for 5 minutes. After the completion of the extraction, filtration with a 140 mesh was performed to collect an extraction residue (tea residue). 200 g of hot water was poured onto the tea residue, followed by extraction for 5 minutes, to collect the tea residue. Again, extraction from the tea residue was performed in the same manner to collect the tea residue.

Thereafter, the tea (tea residue) subjected to the pre-extraction was degraded with an enzyme. 200 g of hot water at 50°C was poured into the tea residue (total amount), and 1 g of protease (product name: PROTIN NY100, available from Daiwa Fine Chemicals Co. Ltd.) was added thereto, followed by reaction in a water bath at 55°C for 3 hours under stirring (300 rpm) with a stirrer. Thereafter, it was held at 95°C for 30 minutes to inactivate the enzyme.

The enzyme-treated solution was heated in the form of a tea liquid mixture without solid-liquid separation. The heat treatment was performed in an autoclave (available from TOMY SEIKO CO., LTD.) using a high-temperature high-pressure fluid at 135°C for 3 hours. The solution after the treatment was filtered with a 140 mesh, to obtain a heat-treated product of tea peptide. Thereafter, it was subjected to freeze drying to obtain a heat-treated product of tea peptide (tea extract) in powder form.

### (3) Preparation of heat-treated product of collagen peptide

Collagen peptide (MDP1, available from Nippi, Incorporated) was added to distilled water to give 250 mg/mL and was put into an autoclave (available from TOMY SEIKO CO., LTD.), followed by high-temperature high-pressure treatment at 135°C and 0.31 MPa for 10 hours, to obtain a heat-treated product of collagen peptide.

### (4) Preparation of heat-treated product of whey peptide

### 30 ml of distilled water was added to 3 g of whey peptide

PeptigenIF-3090 (available from Arla Foods amba with an average molecular weight of 300 to 400), whey peptide with an average molecular weight of 440, or casein peptide CU2500A (available from Morinaga Milk Industry Co.,Ltd. with an average molecular weight of 375) and was put into an autoclave (available from TOMY SEIKO CO., LTD.), followed by high-temperature high-pressure treatment at 135°C and 0.31 MPa for 3 hours, to prepare a heat-treated product of whey peptide.

### <Sample beverages 39 to 43>

Sample beverages 39 to 43 were prepared by adding cycloprolyl threonine (chemically synthesized products) or the heat-treated products of various peptides to a commercially available tea beverage (green tea). 0.021 mL of a cycloprolyl threonine solution with a stock solution concentration of 1 mg/mL was added, and the heat-treated products of various peptides prepared by the aforementioned method were added.

### <Sensory evaluation test>

Sample beverages 1 to 43 were subjected to a sensory evaluation by three expert panelists. Specifically, scoring was performed by the expert panelists based on the following criteria, and the average scores are shown in Tables 1 to 3. Beverages with the average score of 3 or more were determined to be favorable.

### (Criteria for sensory evaluation)

Score 5: Very favorably drinkable, not only with effects due to cycloprolyl threonine remarkable and unpleasant bitterness derived from epigallocatechin gallate removed, but also with complex depth given to aftertaste.

Score 4: Favorably drinkable, with effects due to cycloprolyl threonine present and unpleasant bitterness in aftertaste derived from epigallocatechin gallate improved.

Score 3: Drinkable, with lingering bitterness derived from epigallocatechin gallate improved by cycloprolyl threonine.

Score 2: Not favorably drinkable due to bitterness, even with lingering bitterness derived from epigallocatechin gallate slightly masked.

Score 1: Not favorably drinkable due to excessively strong lingering monotonical bitterness derived from epigallocatechin gallate or unpleasant astringent taste derived from cycloprolyl threonine.

Score 0: No problem, with no lingering bitterness derived from epigallocatechin gallate sensed.

**[Table 1]**

| | Cyclo(Pro-Thr) (stock solution concentration: 1 mg/mL) | Epigallocatechin gallate (stock solution concentration: 10 mg/mL) | Water | Total amount | Cyclo(Pro-Thr) concentration | Epigallocatechin gallate concentration | Sensory evaluation score |
|---|---|---|---|---|---|---|---|
| | (mL) | (mL) | (mL) | (mL) | (mg/100mL) | (mg/100mL) | |
| 1 | 0.0 | 0.01 | 100.0 | 100 | 0.0 | 0.1 | 0 |
| 2 | 0.0 | 0.05 | 100.0 | 100 | 0.0 | 0.5 | 1 |
| 3 | 0.0 | 0.50 | 99.5 | 100 | 0.0 | 5 | 1 |
| 4 | 0.0 | 1.00 | 99.0 | 100 | 0.0 | 10 | 1 |
| 5 | 0.0 | 1.50 | 98.5 | 100 | 0.0 | 15 | 1 |
| 6 | 0.0021 | 0.00 | 100.0 | 100 | 0.0021 | 0.0 | 0 |
| 7 | 0.0021 | 0.01 | 100.0 | 100 | 0.0021 | 0.1 | 0 |
| 8 | 0.0021 | 0.05 | 99.9 | 100 | 0.0021 | 0.5 | 3 |
| 9 | 0.0021 | 0.50 | 99.5 | 100 | 0.0021 | 5 | 4 |
| 10 | 0.0021 | 1.00 | 99.0 | 100 | 0.0021 | 10 | 3 |
| 11 | 0.0021 | 2.00 | 98.0 | 100 | 0.0021 | 20 | 1 |
| 12 | 0.0053 | 0.10 | 99.9 | 100 | 0.0053 | 1 | 5 |
| 13 | 0.0053 | 0.80 | 99.2 | 100 | 0.0053 | 8 | 4 |
| 14 | 0.0053 | 1.50 | 98.5 | 100 | 0.0053 | 15 | 3 |
| 15 | 0.0053 | 2.00 | 98.0 | 100 | 0.0053 | 20 | 1 |
| 16 | 0.021 | 0.05 | 99.9 | 100 | 0.021 | 0.5 | 4 |
| 17 | 0.021 | 0.50 | 99.5 | 100 | 0.021 | 5 | 5 |
| 18 | 0.021 | 1.00 | 99.0 | 100 | 0.021 | 10 | 4 |
| 19 | 0.021 | 1.50 | 98.5 | 100 | 0.021 | 15 | 3 |
| 20 | 0.021 | 2.00 | 98.0 | 100 | 0.021 | 20 | 1 |
| 21 | 0.053 | 0.00 | 99.9 | 100 | 0.053 | 0.0 | 1 |
| 22 | 0.053 | 0.10 | 99.8 | 100 | 0.053 | 1 | 5 |
| 23 | 0.053 | 0.80 | 99.1 | 100 | 0.053 | 8 | 4 |
| 24 | 0.053 | 1.50 | 98.4 | 100 | 0.053 | 15 | 3 |
| 25 | 0.053 | 2.00 | 97.9 | 100 | 0.053 | 20 | 2 |
| 26 | 0.32 | 0.01 | 99.7 | 100 | 0.32 | 0.1 | 1 |
| 27 | 0.32 | 0.05 | 99.6 | 100 | 0.32 | 0.5 | 3 |
| 28 | 0.32 | 0.50 | 99.2 | 100 | 0.32 | 5 | 3 |
| 29 | 0.32 | 1.00 | 98.7 | 100 | 0.32 | 10 | 3 |
| 30 | 0.32 | 1.50 | 98.2 | 100 | 0.32 | 15 | 3 |
| 31 | 1.0 | 0.00 | 99.0 | 100 | 1.0 | 0.0 | 1 |
| 32 | 1.0 | 0.05 | 99.0 | 100 | 1.0 | 0.5 | 1 |
| 33 | 1.0 | 0.50 | 98.5 | 100 | 1.0 | 5 | 2 |
| 34 | 1.0 | 1.00 | 98.0 | 100 | 1.0 | 10 | 2 |

**[Table 2]**

| | Content of various extracts | Epigallocatechin gallate (stock solution concentration: 10 mg/mL) | Total amount | Cyclo(Pro-Thr) concentration | Epigallocatechin gallate concentration | Sensory evaluation score |
|---|---|---|---|---|---|---|
| | | (mL) | (mL) | (mg/100mL) | (mg/100mL) | |
| 35 | Heat-treated product of tea peptide: 50mg | 0.5 | 100 | 0.026 | 5.0 | 4 |
| 36 | Heat-treated product of soybean peptide: 20mg | 0.5 | 100 | 0.022 | 5.0 | 5 |
| 37 | Heat-treated product of collagen peptide: 200mg | 0.5 | 100 | 0.028 | 5.0 | 4 |
| 38 | Heat-treated product of whey peptide: 15 mg | 0.5 | 100 | 0.025 | 5.0 | 5 |

**[Table 3]**

| | Cyclo(Pro-Thr) (stock solution concentration: 1 mg/mL) or content of various extracts | Tea beverage | Total amount | Cyclo(Pro-Thr) concentration | Epigallocatechin gallate concentration | Sensory evaluation score |
|---|---|---|---|---|---|---|
| | | (mL) | (mL) | (mg/100mL) | | |
| 39 | Cyclo(Pro-Thr) stock solution: 0.2 mL | 99.98 | 100 | 0.021 | 6.5 | 5 |
| 40 | Heat-treated product of tea peptide: 50 mg | 100 | 100 | 0.026 | 6.5 | 5 |
| 41 | Heat-treated product of soybean peptide: 20 mg | 100 | 100 | 0.022 | 6.5 | 5 |
| 42 | Heat-treated product of collagen peptide: 200 mg | 100 | 100 | 0.028 | 6.5 | 4 |
| 43 | Heat-treated product of whey peptide: 15 mg | 100 | 100 | 0.025 | 6.5 | 4 |

As shown in Table 1, it turned out that beverages with the content of epigallocatechin gallate and the content of cycloprolyl threonine falling within the ranges of the present invention all had a sensory evaluation score of 3 or more and had excellent drinkability with lingering unpleasant bitterness derived from epigallocatechin gallate in the beverages reduced. Further, as shown in Table 2, the effects of the present invention could be obtained also in the case of using heat-treated products of various peptides to adjust the content of cycloprolyl threonine. Further, as shown in Table 3, the effects of the present invention could be obtained also in the case of adding chemically synthesized cycloprolyl threonine or heat-treated products of various peptides to a commercially available tea beverage to adjust the content of epigallocatechin gallate and the content of cycloprolyl threonine. Accordingly, it was revealed that the present invention could achieve a food or beverage product having favorable taste, with lingering unpleasant bitterness unique to epigallocatechin gallate reduced, by adjusting the content of epigallocatechin gallate and the content of cycloprolyl threonine in the food or beverage product to the ranges of the present invention.

### INDUSTRIAL APPLICABILITY

The present invention provides new means for preparing a food or beverage product having good taste with lingering unpleasant bitterness derived from epigallocatechin gallate in the food or beverage product reduced and therefore has high industrial applicability.

## Claims

1. A food or beverage product comprising epigallocatechin gallate and cycloprolyl threonine, wherein
a content of epigallocatechin gallate is 0.40 to 16 mg/100 mL and
a content of cycloprolyl threonine is 0.0020 to 0.35 mg/100 mL.

2. The food or beverage product according to claim 1, wherein the content of epigallocatechin gallate is 0.90 to 11 mg/100 mL.

3. The food or beverage product according to claim 1 or 2, wherein the content of cycloprolyl threonine is 0.0050 to 0.055 mg/100 mL.

4. The food or beverage product according to claim 1, wherein
the content of epigallocatechin gallate is 0.40 to 11 mg/100 mL,
the content of cycloprolyl threonine is 0.0020 to 0.055 mg/100 mL, and
the content of epigallocatechin gallate (mg/100 mL) (X) and the content of cycloprolyl threonine (mg/100 mL) (Y) satisfy Y ≥ 0.0034 × X^{-1.106} and Y ≥ 0.00009 × X^{1.7421}.

5. The food or beverage product according to any one of claims 1 to 4, wherein the cycloprolyl threonine is added as a heat-treated product of animal and plant-derived peptide.

6. The food or beverage product according to claim 5, wherein the heat-treated product of animal or plant-derived peptide is obtained from soybean peptide, tea peptide, whey peptide, or collagen peptide.

7. The food or beverage product according to any one of claims 1 to 6, wherein the food or beverage product is a tea beverage.

8. The food or beverage product according to any one of claims 1 to 7, wherein the food or beverage product is packaged in a container.

9. A method for producing a food or beverage product, comprising:
step (a) of adding epigallocatechin gallate to adjust the content of epigallocatechin gallate in the food or beverage product to 0.40 to 16 mg/100 mL; and
step (b) of adding cycloprolyl threonine to adjust the content of cycloprolyl threonine in the food or beverage product to 0.0020 to 0.35 mg/100 mL.

10. The production method according to claim 9, wherein the content of epigallocatechin gallate adjusted in step (a) is 0.90 to 11 mg/100 mL.

11. The production method according to claim 9 or 10, wherein the content of cycloprolyl threonine adjusted in step (b) is 0.0050 to 0.055 mg/100 mL.

12. A method for reducing lingering unpleasant astringent taste of epigallocatechin gallate in a food or beverage product, comprising:
step (a) of adding epigallocatechin gallate to adjust the content of epigallocatechin gallate in the food or beverage product to 0.40 to 16 mg/100 mL; and
step (b) of adding cycloprolyl threonine to adjust the content of cycloprolyl threonine in the food or beverage product to 0.0020 to 0.35 mg/100 mL.

13. The production method according to claim 12, wherein the content of epigallocatechin gallate adjusted in step (a) is 0.90 to 11 mg/100 mL.

14. The production method according to claim 12 or 13, wherein the content of cycloprolyl threonine adjusted in step (b) is 0.0050 to 0.055 mg/100 mL.

## Patentansprüche

1. Ein Nahrungsmittel- oder Getränkeprodukt, umfassend Epigallocatechingallat und Cycloprolylthreonin, wobei
ein Gehalt an Epigallocatechingallat 0,40 bis 16 mg/100 mL beträgt und
ein Gehalt an Cycloprolylthreonin 0,0020 bis 0,35 mg/100 mL beträgt.

2. Das Nahrungsmittel- oder Getränkeprodukt nach Anspruch 1, wobei der Gehalt an Epigallocatechingallat 0,90 bis 11 mg/100 mL beträgt.

3. Das Nahrungsmittel- oder Getränkeprodukt nach Anspruch 1 oder 2, wobei der Gehalt an Cycloprolylthreonin 0,0050 bis 0,055 mg/100 mL beträgt.

4. Das Nahrungsmittel- oder Getränkeprodukt nach Anspruch 1, wobei der Gehalt an Epigallocatechingallat 0,40 bis 11 mg/100 mL beträgt,
der Gehalt an Cycloprolylthreonin 0,0020 bis 0,055 mg/100 mL beträgt, und
der Gehalt an Epigallocatechingallat (mg/100 mL) (X) und der Gehalt an Cycloprolylthreonin (mg/100 mL) (Y) Y ≥ 0,0034 × X^{-1,101} und Y ≥ 0,00009 × X^{1,7421} erfüllen.

5. Das Nahrungsmittel- oder Getränkeprodukt nach einem der Ansprüche 1 bis 4, wobei das Cycloprolylthreonin als wärmebehandeltes Produkt aus Peptid, abgeleitet von Tier und Pflanze, zugesetzt wird.

6. Das Nahrungsmittel- oder Getränkeprodukt nach Anspruch 5, wobei das wärmebehandelte Produkt aus Peptid, abgeleitet von Tier und Pflanze, aus Sojabohnenpeptid, Teepeptid, Molkepeptid oder Kollagenpeptid erhalten wird.

7. Das Nahrungsmittel- oder Getränkeprodukt nach einem der Ansprüche 1 bis 6, wobei das Nahrungsmittel- oder Getränkeprodukt ein Teegetränk ist.

8. Das Nahrungsmittel- oder Getränkeprodukt nach einem der Ansprüche 1 bis 7, wobei das Nahrungsmittel- oder Getränkeprodukt in einem Behälter verpackt ist.

9. Ein Verfahren zur Herstellung eines Nahrungsmittel- oder Getränkeprodukts, umfassend:
Schritt (a) die Zugabe von Epigallocatechingallat, um den Gehalt an Epigallocatechingallat in dem Nahrungsmittel- oder Getränkeprodukt auf 0,40 bis 16 mg/100 mL einzustellen; und
Schritt (b) die Zugabe von Cycloprolylthreonin, um den Gehalt an Cycloprolylthreonin in dem Nahrungsmittel- oder Getränkeprodukt auf 0,0020 bis 0,35 mg/100 mL einzustellen.

10. Das Herstellungsverfahren nach Anspruch 9, wobei der in Schritt (a) eingestellte Gehalt an Epigallocatechingallat 0,90 bis 11 mg/100 mL beträgt.

11. Das Herstellungsverfahren nach Anspruch 9 oder 10, wobei der in Schritt (b) eingestellte Gehalt an Cycloprolylthreonin 0,0050 bis 0,055 mg/100 mL beträgt.

12. Ein Verfahren zur Verringerung des anhaltenden unangenehmen adstringierenden Geschmacks von Epigallocatechingallat in einem Nahrungsmittel- oder Getränkeprodukt, umfassend:
Schritt (a) die Zugabe von Epigallocatechingallat, um den Gehalt an Epigallocatechingallat in dem Nahrungsmittel- oder Getränkeprodukt auf 0,40 bis 16 mg/100 mL einzustellen; und
Schritt (b) die Zugabe von Cycloprolylthreonin, um den Gehalt an Cycloprolylthreonin in dem Nahrungsmittel- oder Getränkeprodukt auf 0,0020 bis 0,35 mg/100 mL einzustellen.

13. Das Herstellungsverfahren nach Anspruch 12, wobei der in Schritt (a) eingestellte Gehalt an Epigallocatechingallat 0,90 bis 11 mg/100 mL beträgt.

14. Das Herstellungsverfahren nach Anspruch 12 oder 13, wobei der in Schritt (b) eingestellte Gehalt an Cycloprolylthreonin 0,0050 bis 0,055 mg/100 mL beträgt.

## Revendications

1. Produit de type aliment ou boisson comprenant du gallate d'épigallocatéchine et de la cycloprolyl thréonine, dans lequel
une teneur en gallate d'épigallocatéchine est de 0,40 à 16 mg/100 mL et
une teneur en cycloprolyl thréonine est de 0,0020 à 0,35 mg/100 mL.

2. Produit de type aliment ou boisson selon la revendication 1, dans lequel la teneur en gallate d'épigallocatéchine est de 0,90 à 11 mg/100 mL.

3. Produit de type aliment ou boisson selon la revendication 1 ou 2, dans lequel la teneur en cycloprolyl thréonine est de 0,0050 à 0,055 mg/100 mL.

4. Produit de type aliment ou boisson selon la revendication 1, dans lequel
la teneur en gallate d'épigallocatéchine est de 0,40 à 11 mg/100 mL,
la teneur en cycloprolyl thréonine est de 0,0020 à 0,055 mg/100 mL et
la teneur en gallate d'épigallocatéchine (mg/100 mL) (X) et la teneur en cycloprolyl thréonine (mg/100 mL) (Y) satisfont Y ≥ 0,0034 x X^{-1,106} et Y ≥ 0,00009 x X^{1,7421}.

5. Produit de type aliment ou boisson selon l'une quelconque des revendications 1 à 4, dans lequel la cycloprolyl thréonine est ajoutée en tant que produit traité thermiquement de peptide d'origine animale et végétale.

6. Produit de type aliment ou boisson selon la revendication 5, dans lequel le produit traité thermiquement de peptide d'origine animale ou végétale est obtenu à partir de peptide de soja, de peptide de thé, de peptide de lactosérum ou de peptide de collagène.

7. Produit de type aliment ou boisson selon l'une quelconque des revendications 1 à 6, dans lequel le produit de type aliment ou boisson est une boisson de type thé.

8. Produit de type aliment ou boisson selon l'une quelconque des revendications 1 à 7, dans lequel le produit de type aliment ou boisson est conditionné dans un récipient.

9. Procédé pour produire un produit de type aliment ou boisson, comprenant:
l'étape (a) d'ajout de gallate d'épigallocatéchine pour ajuster la teneur en gallate d'épigallocatéchine dans le produit de type aliment ou boisson à 0,40 à 16 mg/100 mL; et
l'étape (b) d'ajout de cycloprolyl thréonine pour ajuster la teneur en cycloprolyl thréonine dans le produit de type aliment ou boisson à 0,0020 à 0,35 mg/100 mL.

10. Procédé de production selon la revendication 9, dans lequel la teneur en gallate d'épigallocatéchine ajustée à l'étape (a) est de 0,90 à 11 mg/100 mL.

11. Procédé de production selon la revendication 9 ou 10, dans lequel la teneur en cycloprolyl thréonine ajustée à l'étape (b) est de 0,0050 à 0,055 mg/100 mL.

12. Procédé pour réduire le goût astringent désagréable tenace de gallate d'épigallocatéchine dans un produit de type aliment ou boisson, comprenant:
l'étape (a) d'ajout de gallate d'épigallocatéchine pour ajuster la teneur en gallate d'épigallocatéchine dans le produit de type aliment ou boisson à 0,40 à 16 mg/100 mL; et
l'étape (b) d'ajout de cycloprolyl thréonine pour ajuster la teneur en cycloprolyl thréonine dans le produit de type aliment ou boisson à 0,0020 à 0,35 mg/100 mL.

13. Procédé de production selon la revendication 12, dans lequel la teneur en gallate d'épigallocatéchine ajustée à l'étape (a) est de 0,90 à 11 mg/100 mL.

14. Procédé de production selon la revendication 12 ou 13, dans lequel la teneur en cycloprolyl thréonine ajustée à l'étape (b) est de 0,0050 à 0,055 mg/100 mL.
